# EUROPÄISCHE PATENTSCHRIFT

(11) **EP 2 668 965 B1**
(45) Veröffentlichungstag und Bekanntmachung des Hinweises auf die Patenterteilung: **24.02.2016**
(21) Anmeldenummer: 13002680.0
(22) Anmeldetag: 23.05.2013
(51) Int. Cl.: A61L 9/16, A61L 9/014, B01D 46/00, B01D 46/42, B01D 46/44, F24F 13/28

(54) **Mobile Vorrichtung zum Filtern von Luft**
Mobile device for filtering air
Dispositif mobile pour la filtration de l'air

(30) Priorität: 30.05.2012 DE 102012010688; 18.01.2013 DE 102013100507
(43) Veröffentlichungstag der Anmeldung: 04.12.2013
(73) Patentinhaber: deconta GmbH, 46419 Isselburg (DE)
(72) Erfinder: Kamperschroer, Peter, 46485 Wesel (DE); Hemming, Martin, 46485 Wesel (DE)
(74) Vertreter: Rätsch, Caroline

(56) Entgegenhaltungen:
- EP-A2- 0 162 022
- WO-A2-2009/028767
- DE-B- 1 143 622
- US-A- 5 893 939
- US-A1- 2002 066 371

## Beschreibung

Die Erfindung betrifft eine mobile Vorrichtung zum Filtern von Luft, mit einem Gehäuse, das eine flächenförmig gestaltete Frontseite besitzt, in die ein Flächenfilter eingebaut ist, sowie einem Lüfter innerhalb des Gehäuses, der einen Strom ungefilterter Luft durch das Flächenfilter erzeugt und durch eine an anderer Stelle des Gehäuses befindliche Auslassöffnung gefiltert ausbläst, wobei mit dem Gehäuse ein den Ansaugbereich des Flächenfilters stromparallel deckendes Fenster verbunden ist, durch das im Ansaugbereich sich abspielende oder durchgeführte Vorgänge beobachtbar sind, wobei das Fenster in Form eines Deckels schwenkbar mit dem Gehäuse verbunden ist.

Bekannt ist eine solche Vorrichtung gemäß Oberbegriff des Anspruchs 1 aus der US 2002/0066371 A1. Die in dieser Schrift beschriebene Vorrichtung besitzt ein Gehäuse aus einem durchsichtigen Material, wobei das Innere des Gehäuses betrachtet werden kann. Die luftdurchlässige Frontseite ist schwenkbar. Ein Flächenfilter und ein Luflauslass befinden sich an der Rückseite des Gehäuses, während der Lufteinlass an der Vorderseite des Gehäuses vorgesehen ist.

Bekannt ist im weiterhin ein tragbares Raumfilter aus der US 3 523 409 A. Beide Hauptseiten des quaderförmigen Gehäuses sind offen und mit herausnehmbaren Filterscheiben ganzflächig besetzt. In das Gehäuse ist ein elektrisch angetriebener Exhaustor eingebaut, der Luft durch die Filterscheiben ansaugt und über eine Öffnung In der Topseite des Gehäuses ausbläst. Das Gerät soll von einem Zimmer zum anderen innerhalb eines Gebäudes getragen werden können, so dass Insbesondere der dort vorhandene Staubteil der Raumluft aufgefangen werden kann.

Bei beiden vorgenannten Geräten handelt es sich um Vorrichtungen, die als tragbare Geräte Raumluft ansaugen und gefiltert auslassen.

DE 11 43 622 B beschreibt ein Luftreinigungsfilter zum Einbau in ein Gebäude-Fenster. Hierbei befindet sich an einer Fensterscheibe außen einstückig mit dieser ein Filtergehäuse, in dem zahlreiche, aufrecht hintereinander gestellte Filterplatten stehen. Das Gehäuse selbst ist aus durchsichtigem, thermoplastischem Kunststoff hergestellt. Es handelt sich hier nicht um eine mobile Vorrichtung. Das Fenster, gebildet durch einen Teil des Gehäuses, dient nicht dazu, den im Bereich des Gebäudefensters liegenden Teil (da wo "Lufteintritt" steht) zu beobachten, sondern nur dazu, den durch den Filter besetzten Bereich luftzugänglich zu machen. Die in den genannten Raum gelangende Luft wird um 90° umgelenkt.

EP 0 162 022 A2 betrifft eine Filtereinheit insbesondere für Fahrzeug-Klimaanlagen, die vollständig in einen Lufteinlass eingebaut werden kann. Eine Zustandsbeobachtung der individuellen Filterelemente kann durch eine beobachtbare Membran (22) gemäß Figur 4 erfolgen. Wesentlich ist auch, dass das Gehäuse der Vorrichtung gemäß EP 0 162 022 A2 insgesamt undurchsichtig ist.

Die Aufgabenstellung gegenüber dem Stand der Technik geht davon aus, dass bei manchen Manipulationen, die von Personen durchgeführt werden, sich Geruchs- und Staubquellen einstellen, die in unmittelbarer Nähe der handelnden Person auftreten können. Als Beispiel hierfür seien genannt:
- Wechsel des Stomabeutels (Stoma = künstliche Darmöffnung; extreme Geruchsbelästigung durch gärenden Darminhalt);
- Löten und niedrig schmelzendes Lot mit Qualmentwicklung;
- Zwiebel- und Knoblauchschälen.

Für die Erfindung stellt sich damit die Aufgabe, eine tragbare Vorrichtung zur Filterung von Raumluft anzugeben, die bei derartigen Arbeiten eine ungestörte Beobachtung der Arbeit ermöglicht.

Diese Aufgabe wird gelöst bei einer mobilen Vorrichtung gemäß dem Oberbegriff des Anspruch 1, bei der der Deckel eine gerahmte, durchsichtige Scheibe umfasst.

Der bewegliche Deckel spielt eine wesentliche Rolle, da er sowohl das Feld, aus dem die Luft abgesogen wird, umschließen kann, als auch den Beobachtungsverlauf wesentlich vereinfacht. Das Fenster in Form eines Deckels ist schwenkbar mit dem Gehäuse verbunden, wobei vorzugsweise ein Scharnier zum Schwenken des Fensters an der Oberseite des Gehäuses angebracht ist.

Diese Scheibe kann auch auf einem Teil ihrer transparenten Fläche als Lupe gestaltet sein, wobei eine einfache Form der Lupe eine Fresnel-Linse sein kann.

Vorzugsweise trägt der Deckel wenigstens an einer seiner freien Kanten eine starre Schürze oder einen flexiblen Vorhang.

Erleichtert wird die Arbeit, wenn am Gehäuse und/oder am Fenster eine Beleuchtungsvorrichtung angebracht ist. Derartige Beleuchtungsvorrichtungen sind an sich bekannt (vergl. US 5 893 939 A).

Das Flächenfilter an der Frontseite der Vorrichtung kann ein Geruchsfilter, insbesondere ein Aktivkohlenfilter sein, aber auch ein kombinierter Partikelund Geruchsfilter, sein.

Die Auslassöffnung für den gefilterten Luftstrom befindet sich vorzugsweise, ähnlich wie bei dem eingangs genannten Stand der Technik in einer gegenüber der Frontseite angeordneten Gehäuseseite.

Das Flächenfilter wird als plattenartiges Element in einer Führung herausnehmbar gehalten, um ein schnelles Auswechseln zu ermöglichen.

Vorzugsweise wird der Lüfter durch einen Elektromotor angetrieben, der mit einem Akkumulator gespeist wird, der sich innerhalb des Gehäuses befindet. Anstelle dieser Anordnung kann aber auch eine externe Stromquelle für den Elektromotor eingesetzt werden.

Um die Vorrichtung ohne besondere Bemühungen im Bereich des Arbeitsfeldes zu halten, kann diese mit einem Tragriemen versehen oder mit einem Ständer verbunden sein, wobei letzterer auch verfahrbar sein kann.

Ein Ausführungsbeispiel der Erfindung ist in der Zeichnung dargestellt. Die Figuren zeigen im Einzelnen:
- Fig. 1: eine mobile Vorrichtung zum Filtern von Luft, perspektivisch von der Seite gesehen;
- Fig. 2: die Vorrichtung gem. Fig. 1 mit aufgeklapptem Deckel, in Arbeitsstellung;
- Fig. 3: die Vorrichtung in der Stellung gem. Fig. 2, mit Blick auf den Flächenfilter;
- Fig. 4: die Vorrichtung gem. Fig.1, von der Rückseite gesehen;
- Fig. 5: eine schematisierte Darstellung der Vorrichtung, von der Seite gesehen;
- Fig. 6: einen Blick auf die Ansaugseite des Gehäuses;
- Fig. 7: eine Ausführung mit Tragriemen;
- Fig. 8: ein schematisiertes Einsatzbeispiel der Vorrichtung bei Wechsel eines Stomabeutels.

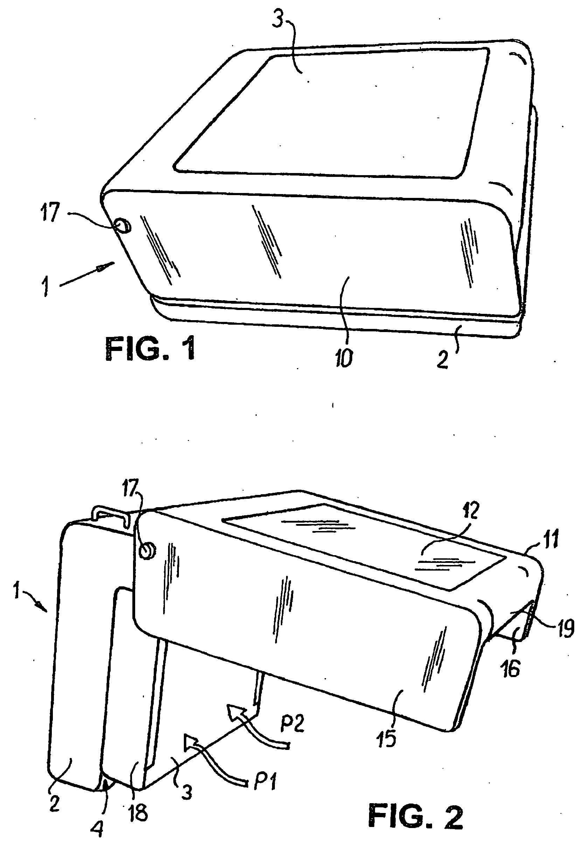

In den Figuren 1 bis 4 ist eine Vorrichtung 1 zum Filtern von Luft dargestellt, deren Abmessungen und Gewicht so bemessen sind, dass sie insbesondere von Arbeitsplatz zu Arbeitsplatz tragbar ist, aber auch an einem Tragriemen vor dem Körper eines Menschen gehalten werden kann.

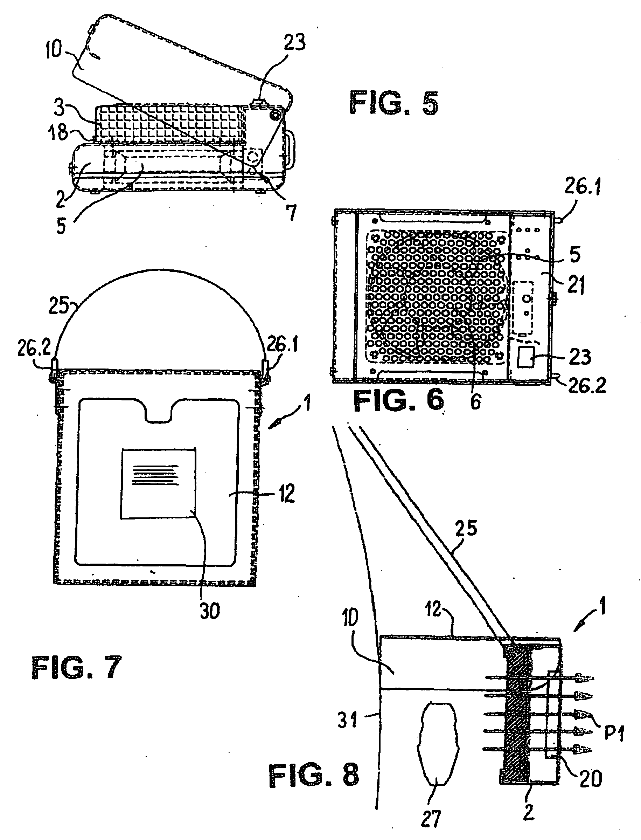

Die Vorrichtung 1 besitzt ein quaderförmiges Gehäuse 2, das im einsatzbereiten Zustand in Seitenansicht L-förmig gestaltet ist, wobei der lange Schenkel des L ein Flächenfilter 3 vor der flächenförmig gestalteten Frontseite 4 trägt. Innerhalb des Gehäuses 2 und hinter dem Flächenfilter 3 befinden sich ein Lüfterrad 5 (vgl. Fig. 5 und 6), das von einem in der Nabe des Lüfterrads 5 angeordneten Elektromotor 6 angetrieben wird. Letzteres erhält seine Energie über einen Akkumulator 7.

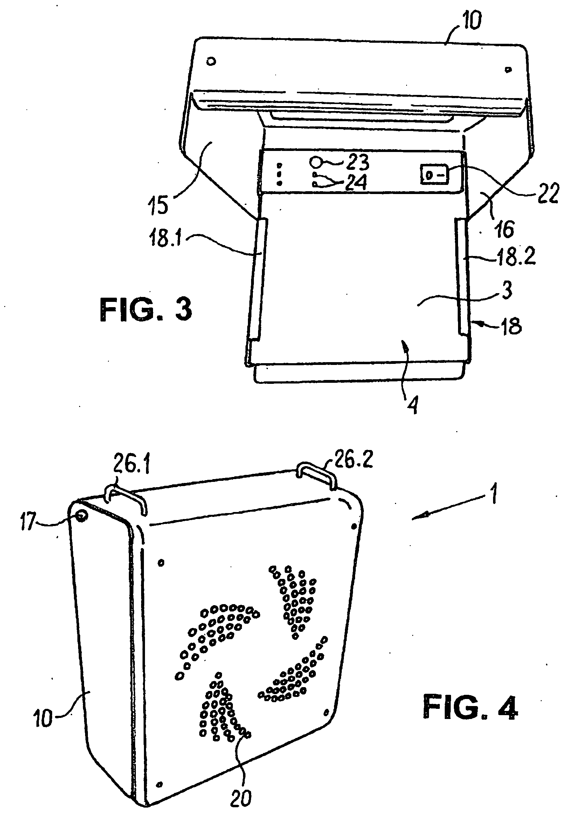

Wesentliches Teil der Vorrichtung 1 ist ein Deckel 10, der um etwa 90° gegenüber seinem geschlossenen Zustand (vgl. Fig. 1) in Bezug auf das Gehäuse 2 aufklappbar ist (vgl. Fig. 2). Der Deckel 10, dessen Gestaltung insbesondere aus den Figuren 2 und 3 ersichtlich ist, besitzt eine Deckel-Oberseite 11, die eine rahmenartige Gestalt hat und eine rechteckige Öffnung besitzt, in der eine durchsichtige Scheibe 12 aus Silikat- oder Polymer- Glas gehalten ist. Innerhalb des Scheibenbereiches, d.h. wenigstens auf einem Teil der transparenten Fläche, kann auch eine Lupe 30, ein Farbfilter oder dergleichen angebracht sein. Die Lupe 30 ist vorzugsweise eine Fresnel-Linse (vgl. Fig. 7).

Die Scheibe 12 erlaubt demnach einen Blick auf den Ansaugbereich 13 der Vorrichtung, so dass Vorgänge im Ansaugbereich 13 zu beobachten sind. Das die Scheibe 12 haltende Rahmenteil 14 setzt sich seitlich in zwei starre Schürzen 15, 16 fort, die senkrecht zum Rahmenteil 14 stehen. Im eingeklappten Zustand des Deckels 10 decken die Schürzen 15, 16 die Seite des Gehäuses ab. Auch ist das Gehäuse 2 am kurzen L-Schenkel auf seinen beiden Seiten mit einem Gelenk 17, hier jeweils ein Schraubbolzen, versehen, das mit je einer Schürze 15, 16 an deren filterseitigen Ende verbunden ist und so das Verschwenken des Deckels ermöglicht.

Zusätzlich sind noch Rastvorrichtungen (nicht dargestellt) vorgesehen, die eine unbeabsichtigte Verstellung des geschlossenen bzw. geöffneten Deckels 10 verhindern. Gegenüberliegend zum Scharnier sind die Schürzen durch einen parallel zur Frontseite 4 verlaufenden Stirnlappen 19 verbunden, der aus dem Rahmenteil 14 herausgebogen ist.

Falls gewünscht, kann anstelle eines an Scharnieren gehaltenen Deckels dieser auch als Kappe aufsetzbar gestaltet sein.

Das Flächenfilter 3 besteht aus einer etwa 30mm dicken Aktivkohle-Masse, die mit Fasern durchsetzt ist und eine quaderförmige Gestalt hat.

Das Flächenfilter 3 dient in erster Linie als Geruchsfilter. Es können jedoch auch andere Filtersubstanzen eingesetzt werden, beispielsweise solche, die Partikel wie Staubteilchen, abfangen oder solche, die gleichzeitig Partikel- und Geruchsfilter sind.

Um jeweilige Flächenfilter 3 nach Erschöpfung seiner Filterkapazität einfach auswechseln zu können, ist vor das Gehäuse 2 eine aus zwei Metallwangen 18.1, 18.2 besehende Führung 18 vorgesehen, in der das Flächenfilter 3 herausnehmbar und verschiebbar gehalten ist.

Das Gehäuse 2, der Deckel 10 und die Schürzen 15,16 mit Stirnlappen 14 bestehen im Ausführungsbeispiel aus beschichtetem Aluminiumblech. Es können aber auch andere Metalle und Legierungen, zum Beispiel Duralumin, sowie Kunststoffe verwendet werden. Anstelle von Metallschürzen können beispielsweise auch flexible Textil- oder Folienmaterialien verwendet werden. Wesentlich erscheint, dass ein möglichst dichter und konzentrierter Luftstrom (vgl. Pfeile P1, P2) zu erzeugen ist, so dass im Ansaugbereich 13 alle nicht erwünschten Partikel und Gasteile durch das Flächenfilter 3 abgezogen werden.

Figuren 5 und 6 lassen in einer schematisierten Ansicht erkennen, dass das Ventilatorrad 5 unmittelbar hinter dem Flächenfilter 3 angeordnet ist. Die gefilterte Luft wird durch das Gehäuse 2 geführt und durch Auslassöffnungen 20, die sich an der Rückseite 21 des Gehäuses 2 befinden, ausgeblasen.

Wenn unterhalb des Deckels 10 gem. Fig. 3 auf die Frontseite 4 geschaut wird, erkennt man, dass der kurze L-Schenkel des Gehäuses 2 an seiner Endseite 21 mit einigen Bedienungselementen ausgerüstet ist (vgl. auch Fig. 6). Mit einem Ein- und Ausschalter 22 kann der Elektromotor 6 geschaltet werden. Verschiedene Kontrolllampen 24 lassen Ladezustand, Filterwechsel und dergleichen erkennen. Ein LED-Beleuchtungskörper 23 beleuchtet den Beobachtungskorb unterhalb der Scheibe 12. Weitere Beleuchtungskörper (nicht dargestellt) können auch an der Unterseite des Deckels 10 oder an der Innenseite der Schürze angebracht werden.

Um die Vorrichtung 1 am Körper tragen zu können, ist ein Tragriemen 25 vorgesehen, der über zwei Ösen 26.1, 26.2 mit dem Gehäuse verbunden ist. Anstelle eines Tragriemens kann die Vorrichtung auch an einem fahrbaren Gestell oder Ständer, ähnlich wie es in Krankenhäusern für das Aufhängen von Transfusionsgefäßen verwendet wird, aufgehängt werden.

Fig. 8 zeigt ein Anwendungsbeispiel der Vorrichtung 1. Die aufgeklappte Vorrichtung 1 hängt an dem Tragriemen 25. Der Deckel 10 stützt sich gegen die Bauchdecke 31 eines Patienten, der einen Stomabeutel 27 auswechselt, wobei der dabei entstehende Geruch störend ist. Durch den Flächenfilter 3 werden die innerhalb des Arbeitsbereiches vorhandenen Gase (P1) abgesaugt und im Flächenfilter 3 gereinigt.

Ähnliche Anwendungsbeispiele sind eingangs genannt, insbesondere Aerosole hervorrufende Lötarbeiten, Beobachtungen von chemischen Reaktionen und dergleichen.

Die äußere Form des Gehäuses 2, die hier als Quaderform dargestellt ist, kann sich an die entsprechenden Arbeitsbereiche anpassen, beispielsweise eine Wölbung aufweisen, die an den menschlichen Körper angepasst ist.

## Patentansprüche

1. Mobile Vorrichtung (1) zum Filtern von Luft, mit einem Gehäuse (2), das eine flächenförmig gestaltete Frontseite (4) besitzt, in die ein Flächenfilter (3) eingebaut ist, sowie mit einem Lüfter (5) innerhalb des Gehäuses (2), der einen Strom ungefilterter Luft durch das Flächenfilter (3) erzeugt und durch eine an anderer Stelle des Gehäuses befindliche Auslassöffnung (20) gefiltert ausbläst, wobei mit dem Gehäuse (2) ein den Ansaugbereich des Flächenfilters (3) stromparallel deckendes Fenster (12) verbunden ist, durch das im Ansaugbereich sich abspielende oder durchgeführte Vorgänge beobachtbar sind, wobei das Fenster (1) in Form eines Deckels (10) schwenkbar mit dem Gehäuse verbunden ist, **dadurch gekennzeichnet, dass** der Deckel (10) eine gerahmte, durchsichtige Scheibe (12) umfasst.

2. Mobile Vorrichtung nach Anspruch 1, **dadurch gekennzeichnet, dass** ein Scharnier zum Schwenken des Fensters an der Oberseite des Gehäuses (2) angebracht ist.

3. Mobile Vorrichtung nach Anspruch 1, **dadurch gekennzeichnet, dass** die Scheibe (12) wenigstens auf einem Teil ihrer transparenten Fläche als Lupe (30), beispielsweise in Form einer Fresnel-Linse, gestaltet ist.

4. Mobile Vorrichtung nach einem der vorhergehenden Ansprüche 1 - 3,
**dadurch gekennzeichnet, dass** der Deckel wenigstens an einer seiner freien Kanten eine starre Schürze (15) oder einen flexiblen Vorhang trägt.

5. Mobile Vorrichtung nach einem der vorhergehenden Ansprüche,
**dadurch gekennzeichnet, dass** am Gehäuse und/oder am Fenster eine Beleuchtungsvorrichtung (23) angebaut ist.

6. Mobile Vorrichtung nach einem der vorhergehenden Ansprüche,
**dadurch gekennzeichnet, dass** das Flächenfilter (3) an der vorderseitigen Flachseite der Vorrichtung ein Geruchsfilter, insbesondere ein Aktivkohlefilter, ist.

7. Mobile Vorrichtung nach einem der vorhergehenden Ansprüche,
**dadurch gekennzeichnet, dass** das Flächenfilter (3) ein Partikel- und Geruchsfilter ist.

8. Mobile Vorrichtung nach Anspruch 1, **dadurch gekennzeichnet, dass** die Auslassöffnung (20) für den gefilterten Luftstrom sich in einer gegenüber der Frontseite angeordneten Gehäuseseite befindet.

9. Mobile Vorrichtung nach einem der vorhergehenden Ansprüche,
**dadurch gekennzeichnet, dass** das Flächenfilter als plattenartiges Element in einer Führung (18) herausnehmbar gehalten ist.

10. Mobile Vorrichtung nach einem der vorhergehenden Ansprüche,
**dadurch gekennzeichnet, dass** der Lüfter durch einen Elektromotor (6) angetrieben ist, der von einem im Gehäuse befindlichen Akkumulator (7) gespeist wird.

11. Mobile Vorrichtung nach einem der vorhergehenden Ansprüche,
**dadurch gekennzeichnet, dass** die Vorrichtung mit einem Tragriemen (25) versehen ist.

12. Mobile Vorrichtung nach einem der vorhergehenden Ansprüche,
**dadurch gekennzeichnet, dass** die Vorrichtung mit einem Ständer verbunden ist.

13. Mobile Vorrichtung nach Anspruch 12, **dadurch gekennzeichnet, dass** der Ständer verfahrbar ist.

## Claims

1. Mobile apparatus (1) for filtering air, having a housing (2) which has a front side (4) of laminar design, into which a surface filter (3) is installed, and having a fan (5) within the housing (2), which fan (5) generates a flow of unfiltered air through the surface filter (3) and blows it out filtered through an outlet opening (20) which is situated at another location of the housing, wherein a window (12) which covers the intake region of the surface filter (3) parallel to the flow is connected to the housing (2), through which window (12) operations which occur or are carried out in the intake region can be observed, wherein the window is connected pivotably to the housing in the form of a cover (10),
**characterized in that** the cover (10) comprises a framed, transparent pane (12).

2. Mobile apparatus according to claim 1, **characterized in that** a hinge for pivoting the window is attached to the upper side of the housing (2).

3. Mobile apparatus according to claim 1, **characterized in that** the pane (12) is configured as a magnifying glass (30) at least on part of its transparent surface, for example in the form of a Fresnel lens.

4. Mobile apparatus according to one of the preceding claims 1 - 3, **characterized in that** the cover carries a rigid apron (15) or a flexible curtain at least on one of its free edges.

5. Mobile apparatus according to one of the preceding claims, **characterized in that** an illumination apparatus (23) is installed on the housing and/or on the window.

6. Mobile apparatus according to one of the preceding claims, **characterized in that** the surface filter (3) on the front flat side of the apparatus is an odour filter, in particular an activated charcoal filter.

7. Mobile apparatus according to one of the preceding claims, **characterized in that** the surface filter (3) is a particle and odour filter.

8. Mobile apparatus according to claim 1, **characterized in that** the outlet opening (20) for the filtered air flow is situated in a housing side which is arranged opposite the front side.

9. Mobile apparatus according to one of the preceding claims, **characterized in that** the surface filter is held removably in a guide (18) as a plate-like element.

10. Mobile apparatus according to one of the preceding claims, **characterized in that** the fan is driven by an electric motor (6) which is fed by a battery (7) which is situated in the housing.

11. Mobile apparatus according to one of the preceding claims, **characterized in that** the apparatus is provided with a carrying belt (25).

12. Mobile apparatus according to one of the preceding claims, **characterized in that** the apparatus is connected to a stand.

13. Mobile apparatus according to claim 12, **characterized in that** the stand is movable.

## Revendications

1. Dispositif mobile (1) pour la filtration de l'air, comprenant un boîtier (2) qui possède un côté avant (4) configuré sous forme plane, dans lequel est incorporé un filtre plat (3) et comprenant un ventilateur (5) à l'intérieur du boîtier (2), lequel produit un flux d'air non filtré à travers le filtre plat (3) et le souffle sous forme filtrée à travers une ouverture de sortie (20) se trouvant à un autre endroit du boîtier, une fenêtre (12) recouvrant la région d'aspiration du filtre plat (3) parallèlement à l'écoulement étant connectée au boîtier (2), à travers laquelle fenêtre des opérations effectuées ou se déroulant dans la région d'aspiration peuvent être observées, la fenêtre (1) étant connectée au boîtier de manière pivotante à la manière d'un couvercle (10), **caractérisé en ce que** le couvercle (10) comprend une vitre transparente (12) encadrée.

2. Dispositif mobile selon la revendication 1, **caractérisé en ce qu'**une charnière est montée sur le côté supérieur du boîtier (2) pour faire pivoter la fenêtre.

3. Dispositif mobile selon la revendication 1, **caractérisé en ce que** la vitre (12) est configurée au moins sur une partie de sa surface transparente sous forme de loupe (30), en particulier sous forme de lentille de Fresnel.

4. Dispositif mobile selon l'une quelconque des revendications précédentes 1-3, **caractérisé en ce que** le couvercle porte au moins au niveau de l'une de ses arêtes libres une jupe rigide (15) ou un rideau flexible.

5. Dispositif mobile selon l'une quelconque des revendications précédentes, **caractérisé en ce qu'**un dispositif d'éclairage (23) est monté sur le boîtier et/ou sur la fenêtre.

6. Dispositif mobile selon l'une quelconque des revendications précédentes, **caractérisé en ce que** le filtre plat (3) sur le côté plat avant du dispositif est un filtre anti-odeur, en particulier un filtre à charbon actif.

7. Dispositif mobile selon l'une quelconque des revendications précédentes, **caractérisé en ce que** le filtre plat (3) est un filtre à particules et anti-odeur.

8. Dispositif mobile selon la revendication 1, **caractérisé en ce que** l'ouverture de sortie (20) pour le flux d'air filtré se trouve dans un côté du boîtier disposé à l'opposé du côté frontal.

9. Dispositif mobile selon l'une quelconque des revendications précédentes, **caractérisé en ce que** le filtre plat sous forme d'élément de type plaque est retenu dans un guide (18) de manière à pouvoir être enlevé.

10. Dispositif mobile selon l'une quelconque des revendications précédentes, **caractérisé en ce que** le ventilateur est entraîné par un moteur électrique (6) qui est alimenté par une batterie (7) se trouvant dans le boîtier.

11. Dispositif mobile selon l'une quelconque des revendications précédentes, **caractérisé en ce que** le dispositif est pourvu d'une courroie de support (25).

12. Dispositif mobile selon l'une quelconque des revendications précédentes, **caractérisé en ce que** le dispositif est connecté à un support.

13. Dispositif mobile selon la revendication 12, **caractérisé en ce que** le support est déplaçable.
